(19) Europäisches Patentamt
European Patent Office
Office européen des brevets

(11) EP 4 083 086 A1

(12) **EUROPEAN PATENT APPLICATION**
published in accordance with Art. 153(4) EPC

(43) Date of publication:
**02.11.2022 Bulletin 2022/44**

(21) Application number: **19957566.3**

(22) Date of filing: **26.12.2019**

(51) International Patent Classification (IPC):
***C08F 220/04*** *(2006.01)* ***A61F 13/53*** *(2006.01)*
***A61L 15/22*** *(2006.01)*

(52) Cooperative Patent Classification (CPC):
**A61F 13/53; A61L 15/22; C08F 220/04;**
Y02W 30/62

(86) International application number:
**PCT/JP2019/051328**

(87) International publication number:
**WO 2021/131003 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kanagawa University**
**Yokohama-shi**
**Kanagawa 221-8686 (JP)**

(72) Inventor: **KIHARA, Nobuhiro**
**Yokohama-shi, Kanagawa 221-8686 (JP)**

(74) Representative: **Ström & Gulliksson AB**
**P.O. Box 4188**
**203 13 Malmö (SE)**

(54) **CROSSLINKED POLYMER COMPOUND AND METHOD FOR PRODUCING SAME, ABSORBENT ARTICLE, PAPER DIAPER, SANITARY ARTICLE, DISPOSAL CONTAINER, AND DISPOSAL METHOD**

(57) Provided are an absorbent article such as a disposable diaper or sanitary article, the absorbent article having a high water absorbing property and being capable of reducing the amount of wastes discharged after use; a cross-linked polymer compound used for same and a method for producing the cross-linked polymer compound; and a treatment container and treatment method for disposing of a disposable diaper, sanitary article, etc. The cross-linked polymer compound according to the present invention includes a skeleton unit shown in Formula (1) below and a skeleton unit shown in Formula (2) below,

Formula (1):

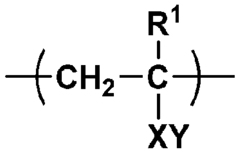

wherein $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is one or more selected from $-COO^-$, $-SO_3^-$, and $-P(=O)(O^-)_2$; and Y is one or more selected from $Li^+$, $Na^+$, and $K^+$, and

Formula (2):

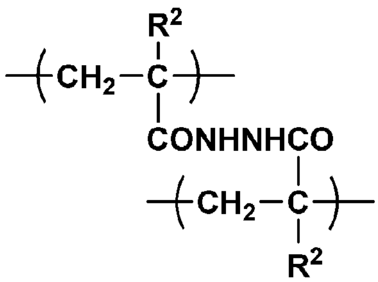

wherein two $R^2$ are each independently one or more selected from hydrogen, an alkyl group, a hydroxy group, an amino group, a mercapto group, a substituted carbo-

EP 4 083 086 A1

nyl group, and any alkyl group having, as a substituent, one or more selected from a hydroxy group, an amino group, a mercapto group, and a substituted carbonyl group.

## Description

TECHNICAL FIELD

The present invention relates to a cross-linked polymer compound and a method for producing the compound, an absorbent article, a disposable diaper, a sanitary article, a treatment container, and a treatment method.

BACKGROUND ART

A copolymer of sodium acrylate with a divinyl crosslinking agent has been used as a water-absorbing material for a disposable diaper or a sanitary article since the copolymer can absorb water up to several hundred to a thousand times its own weight due to high hydrophilicity of the sodium acrylate (e.g., see Patent Document 1).

Meanwhile, disposable diapers or sanitary articles have been consumed in large quantities at nursery schools, hospitals, nursing homes, etc. Used disposable diapers or sanitary articles are usually collected and disposed of, but they tend to be a burden on staff at the nursery schools, the hospitals, the nursing homes, etc. since the disposable diapers or sanitary articles that have absorbed moisture are heavy.

Furthermore, the disposable diapers or the sanitary articles disposed of in large quantities are bulky waste and contain excrement, making them costly for storage and disposal.

Prior Art Documents

Patent Documents

Patent Document 1: Japanese Unexamined Patent Application, Publication No. H11-315147

DISCLOSURE OF THE INVENTION

Problems to be Solved by the Invention

As mentioned above, disposal of the used disposable diapers or the used sanitary articles has become a major social problem, and there is a need for a method for reducing waste from the used disposable diapers or the used sanitary articles.

The present invention has been proposed in view of the above-mentioned related art and an object of the present invention is to provide an absorbent article which has a high water-absorbing property and from which a less amount of wastes is discharged after use, for example, a disposable diaper and a sanitary article; a cross-linked polymer compound for use in the absorbent article; a method for producing the cross-linked polymer compound; and a treatment container and a treatment method for use in treatment of the disposable diaper or the sanitary article.

Means for Solving the Problems

The present inventors conducted extensive studies to solve the above problem. As a result, the present invention has been completed based on findings that a cross-linked polymer compound in which a main chain such as acrylic acid and sodium polyacrylate is cross-linked with diacylhydrazine easily decomposes by treating with an oxidizing agent other than oxygen and, therefore, can be dissolved in water and easily disposed of.

A first aspect of the present invention relates to a cross-linked polymer compound including: a skeleton unit shown in Formula (1) below; and a skeleton unit shown in Formula (2) below,

Formula (1):

[Chem. 1]

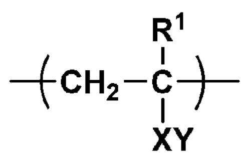

in which $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is one or more selected from $-COO^-$, $-SO_3^-$, and $-P(=O)(O^-)_2$; and Y is one or more selected from $Li^+$, $Na^+$, and $K^+$, and

Formula (2):

[Chem. 2]

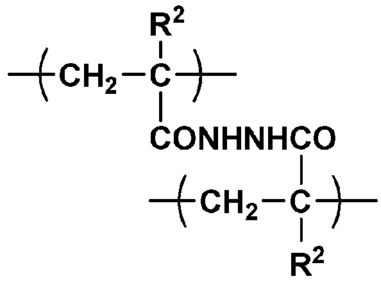

in which two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

A second aspect of the present invention relates to the cross-linked polymer compound as described in the first aspect, consisting of the skeleton unit shown in Formula (1) and the skeleton unit shown in Formula (2), in which a molar ratio of the skeleton unit shown in Formula (1) to the skeleton unit shown in Formula (2) (the skeleton unit shown in Formula (1):the skeleton unit shown in Formula (2)) is 90:10 to 99.9999:0.0001.

A third aspect of the present invention relates to the cross-linked polymer compound as described in the first or second aspect, in which the part $R^1$ in Formula (1) and the two $R^2$ are both H, the part X is $-COO^-$, and the part Y is $Na^+$.

A fourth aspect of the present invention relates to the cross-linked polymer compound as described in any one of the first to third aspects, being a random copolymer.

A fifth aspect of the present invention relates to an absorbent article including: the cross-linked polymer compound as described in any one of the first to fourth aspects, the cross-linked polymer compound being used as a water-absorbing material.

A sixth aspect of the present invention relates to a disposable diaper including: the cross-linked polymer compound as described in any one of the first to fourth aspects, the cross-linked polymer compound being used as a water-absorbing material.

A seventh aspect of the present invention relates to a disposable diaper including: a surface material; a thread for sewing up the surface material; and a water-absorbing material, in which the water-absorbing material is placed into a space enclosed by the surface material sewn up with the thread, the cross-linked polymer compound as described in any one of the first to fourth aspects is used as the water-absorbing material, and a biodegradable compound or an oxidative-degradable compound is used as the thread.

An eighth aspect of the present invention relates to a sanitary article including: the cross-linked polymer compound as described in any one of the first to fourth aspects, the cross-linked polymer compound being used as a water-absorbing material.

A ninth aspect of the present invention relates to a sanitary article including: a surface material; a water-absorbing material; and a thread for sewing up the surface material, in which the water-absorbing material is placed into a space enclosed by the surface material sewn up with the thread, the cross-linked polymer compound as described in any one of the first to fourth aspects is used as the water-absorbing material, and a biodegradable compound or an oxidative-degradable compound is used as the thread.

A tenth aspect of the present invention relates to a treatment container capable of storing: an oxidizing agent other than oxygen; and the disposable diaper as described in the sixth or seventh aspect or the sanitary article as described in the eighth or ninth aspect.

An eleventh aspect of the present invention relates to a treatment method including: putting the disposable diaper as described in the sixth or seventh aspect or the sanitary article as described in the eighth or ninth aspect into the treatment container as described in the tenth aspect which stores an oxidizing agent other than oxygen.

A twelfth aspect of the present invention relates to a method for producing a cross-linked polymer compound, the method including: copolymerizing a monomer shown in Formula (3) below and a monomer shown in Formula (4) below,

Formula (3):

[Chem. 3]

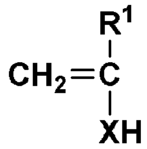

in which $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; and X is one or more selected from $-COO^-$, $-SO_3^-$, and - P (=O)$(O^-)_2$, and

Formula (4):

[Chem. 4]

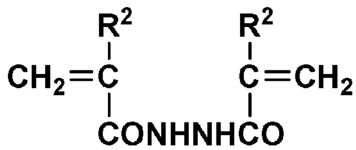

in which two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

Effects of the Invention

According to the present invention, a cross-linked polymer compound in which a main chain such as acrylic acid and sodium polyacrylate is cross-linked with diacylhydrazine easily decomposes by treating with an oxidizing agent other than oxygen and, therefore, can be dissolved in water and disposed of.

PREFERRED MODE FOR CARRYING OUT THE INVENTION

Although embodiments of the present invention will be described in detail below, the present invention is not limited to the embodiments below in any way and can be implemented with modifications as appropriate within a scope that does not change the gist of the present invention.

<<Cross-linked polymer compound>>

A cross-linked polymer compound according to the present embodiment includes a skeleton unit shown in Formula (1) below and a skeleton unit shown in Formula (2) below,

Formula (1):

[Chem. 5]

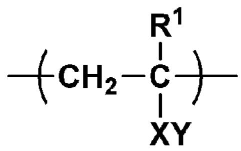

in which $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is one or more selected from $-COO^-$, $-SO_3^-$, and $-P(=O)(O^-)_2$; and Y is one or more selected from $Li^+$, $Na^+$, and $K^+$, and

Formula (2):

[Chem. 6]

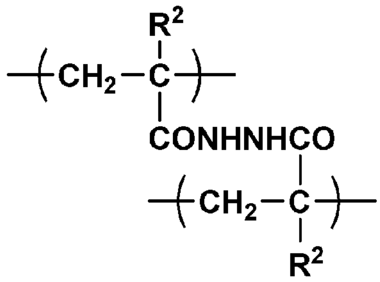

in which two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

Such a cross-linked polymer compound is also represented by Formula (5) below,

Formula (5):

[Chem. 7]

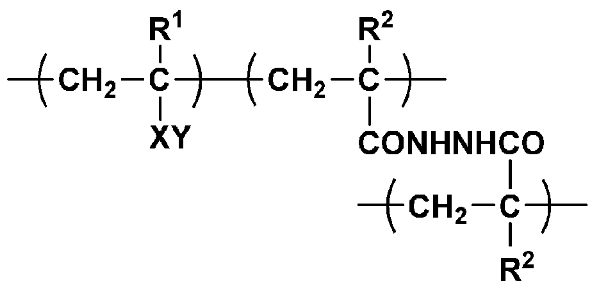

in which $R^1$ and two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is each

independently one or more selected from $-COO^-$, $-SO_3^-$, and - $P(=O)(O^-)_2$; and Y is each independently one or more selected from $Li^+$, $Na^+$, and $K^+$.

As represented by Formula (5), a polymerized chain in the cross-linked polymer compound according to the present embodiment can be considered as having a structure cross-linked with diacylhydrazine (General Formula: $R_1$-CONHNHCO-$R_2$ in which $R_1$ and $R_2$ are each independently any polymerized chain). As represented by Formula (6) below, diacylhydrazine decomposes by reacting with an oxidizing agent other than oxygen to thereby produce carboxylic acid and nitrogen. This reaction allows a diacylhydrazine chain in which the polymerized chain is cross-linked to be cut to thereby release the polymerized chain, which is water-soluble. The polymerized chain is, for example, polyacrylic acid and is extremely low in toxicity for an organism or an environment. Therefore, wastewater containing the polymerized chain which has decomposed to be released can be discharged into sewerage without requiring special treatment.

Formula (6):

[Chem. 8]

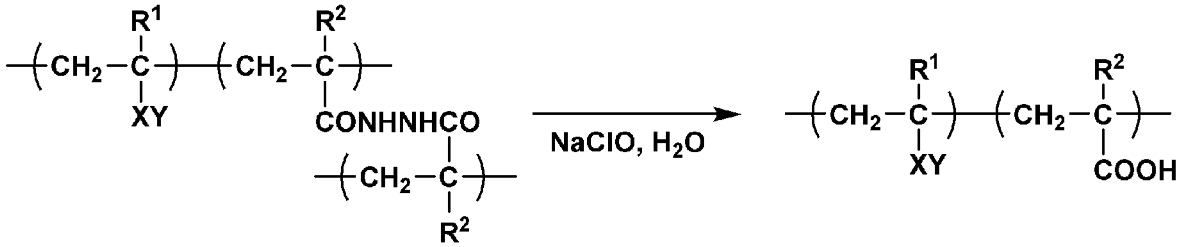


in which $R^1$ and two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is each independently one or more selected from $-COO^-$, $-SO_3^-$, and - $P(=O)(O^-)_2$; and Y is each independently one or more selected from $Li^+$, $Na^+$, and $K^+$.

Furthermore, the cross-linked polymer compound according to the present embodiment has excellent water-absorbing power due to high hydrophilicity of the skeleton unit shown in Formula (1).

The cross-linked polymer compound according to the present embodiment may consist of the skeleton unit shown in Formula (1) and the skeleton unit shown in Formula (2), or may include 50% by mole or less of another skeleton unit other than the skeleton unit shown in Formula (1) and the skeleton unit shown in Formula (2) relative to all skeleton units included in the cross-linked polymer compound.

When the cross-linked polymer compound consists of the skeleton unit shown in Formula (1) and the skeleton unit shown in Formula (2), a ratio of the skeleton unit shown in Formula (1) to the skeleton unit shown in Formula (2) (the skeleton unit shown in Formula (1):the skeleton unit shown in Formula (2)) is not particularly limited, but is preferably 90:10 to 99.9999:0.0001, more preferably 93:7 to 99.99:0.01, further preferably 94:8 to 99.9:0.1, and particularly preferably 97:3 to 99.5:0.5 at a molar ratio. Note that, the expression "A:B to C:D" as used herein means that, when a ratio of the skeleton unit shown in Formula (1) is A or more and C or less, a ratio of the skeleton unit shown in Formula (2) is B or less and D or more, and a sum of the ratio of the skeleton unit shown in Formula (1) and the ratio of the skeleton unit shown in Formula (2) is 100. The above-mentioned range allows the cross-linked polymer compound to appropriately maintain a cross-linked structure while having high hydrophilicity (water-absorbing property) due to a -COONa group.

When the cross-linked polymer compound includes the other skeleton unit other than the skeleton unit shown in (1) and the skeleton unit shown in Formula (2), a monomer constituting the other skeleton unit is not particularly limited. Examples thereof include an anionic unsaturated monomer and salts thereof such as (anhydrous) maleic acid, itaconic acid, cinnamic acid, vinyl sulfonic acid, allyltoluene sulfonic acid, vinyltoluene sulfonic acid, styrenesulfonic acid, 2-(meth)acrylamido-2-methylpropanesulfonic acid, 2-(meth)acryloylethanesulfonic acid, 2-(meth)acryloylpropanesulfonic acid, 2-hydroxyethyl (meth)acryloyl phosphate; a mercaptan group-containing unsaturated monomer; a phenolic hydroxyl group-containing unsaturated monomer; an amide group-containing unsaturated monomer such as (meth)acrylamide, N-ethyl(meth)acrylamide, and N,N-dimethyl(meth)acrylamide; an amino group-containing unsaturated monomer such as N,N-dimethylaminoethyl (meth)acrylate, N,N-dimethylaminopropyl (meth)acrylate, and N,N-dimethylaminopropyl (meth)acrylamide, etc.

When the cross-linked polymer compound includes the other skeleton unit other than the skeleton unit shown in Formula (1) and the skeleton unit shown in Formula (2), a ratio of a skeleton unit other than the skeleton unit shown

in Formula (2) to the skeleton unit shown in Formula (2) (the skeleton unit other than the skeleton unit shown in Formula (2):the skeleton unit shown in Formula (2)) is not particularly limited, but is preferably 90:10 to 99.9999:0.0001, more preferably 93:7 to 99.99:0.01, further preferably 94:8 to 99.9:0.1, and particularly preferably 97:3 to 99.5:0.5 at a molar ratio.

When the cross-linked polymer compound includes the other skeleton unit, the content of the other skeleton unit is not particularly limited as long as it is 50% by mole or less, but may be 45% by mole or less, 40% by mole or less, 35% by mole or less, 30% by mole or less, 27% by mole or less, 25% by mole or less, 22% by mole or less, 20% by mole or less, 15% by mole or less, 12% by mole or less, 10% by mole or less, 9% by mole or less, 8% by mole or less, 7% by mole or less, 6% by mole or less, 5% by mole or less, 4% by mole or less, 3% by mole or less, 2% by mole or less, 1% by mole or less, 0.5% by mole or less, 0.1% by mole or less, 0.05% by mole or less, 0.01% by mole or less, 0.005% by mole or less, 0.001% by mole or less, 0.0005% by mole or less, 0.0001 relative to all skeleton units included in the cross-linked polymer compound. The content of the other skeleton unit may be more than 0% by mole.

As mentioned above, $R^1$ in Formula (1) is not particularly limited as long as it is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group. However, $R^1$ is preferably hydrogen (H) from the viewpoints of a high water-absorbing property and cost of the monomer, etc.

Furthermore, when $R^1$ is an alkyl chain, the number of carbon atoms of the alkyl chain is not particularly limited, but is more preferably 2 or less and more preferably 1.

As mentioned above, X in Formula (1) is not particularly limited as long as it is any one or more of a $-COO^-$ group, a - $SO_3^-$ group, and a $-P(=O)(O^-)_2$ group, but is preferably the $-COO^-$group from the viewpoints of a high water-absorbing property and cost of the monomer, etc.

As mentioned above, Y in Formula (1) is not particularly limited as long as it is any one or more of $Li^+$, $Na^+$, and $K^+$, but is preferably the $Na^+$ group from the viewpoints of a high water-absorbing property and cost of the monomer, etc.

As mentioned above, two $R^2$ in Formula (2) are not particularly limited as long as they are each independently one or more selected from hydrogen, an alkyl group, a hydroxy group, an amino group, a mercapto group, a substituted carbonyl group, and any alkyl group having, as a substituent, the above groups. However, two $R^2$ are both preferably hydrogen (H) from the viewpoints of a high water-absorbing property and a cost of the monomer, etc.

Furthermore, when $R^2$ are alkyl chains, the number of carbon atoms of the alkyl chain are not particularly limited, but are each independently preferably 2 or less and more preferably 1.

Note that, two $R^2$ may be the same as or different from each other as long as they are both those selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

Furthermore, in a polymerized state (state represented by Formula (5)), it is not necessary that $R^1$ is of one type and $R^2$ are of one or two types, and each of $R^1$ and $R^2$ may be of multiple types greater than the above-mentioned number, as long as $R^1$ and $R^2$ are selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group. In other words, the cross-linked polymer compound having such multiple types of $R^1$ and $R^2$ are obtained using multiple types of monomers in which $R^1$ is different from each other each serving as a monomer shown in Formula (3) below and/or multiple types of monomers in which $R^2$ are different from each other each serving as a monomer shown in Formula (4) below.

The cross-linked polymer compound according to the present embodiment may be a block copolymer or a random copolymer in a relationship between the skeleton unit shown in Formula (1) above and the skeleton unit shown in Formula (2) above, but is preferably a random copolymer which is uniformly cross-linked across the polymerized chain from the viewpoint of effectively achieving an effect such as gelation by crosslinking with diacylhydrazine.

<<Method for producing cross-linked polymer compound>>

A method for producing the cross-linked polymer compound according to the present embodiment is a method capable of synthesizing the above-mentioned cross-linked polymer compound. This method for producing the cross-linked polymer compound includes copolymerizing a monomer shown in Formula (3) below and a monomer shown in Formula (4) below,

Formula (3):

[Chem. 9]

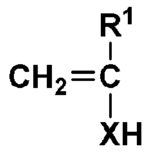

in which $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; and X is one or more selected from $-COO^-$, $-SO_3^-$, and - $P(=O)(O^-)_2$, and

Formula (4):

[Chem. 10]

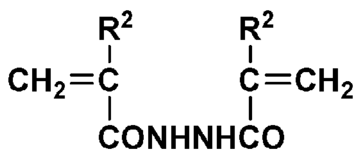

in which two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

A reaction formula of this polymerization reaction is represented by Formula (7) below,

Formula (7):

[Chem. 11]

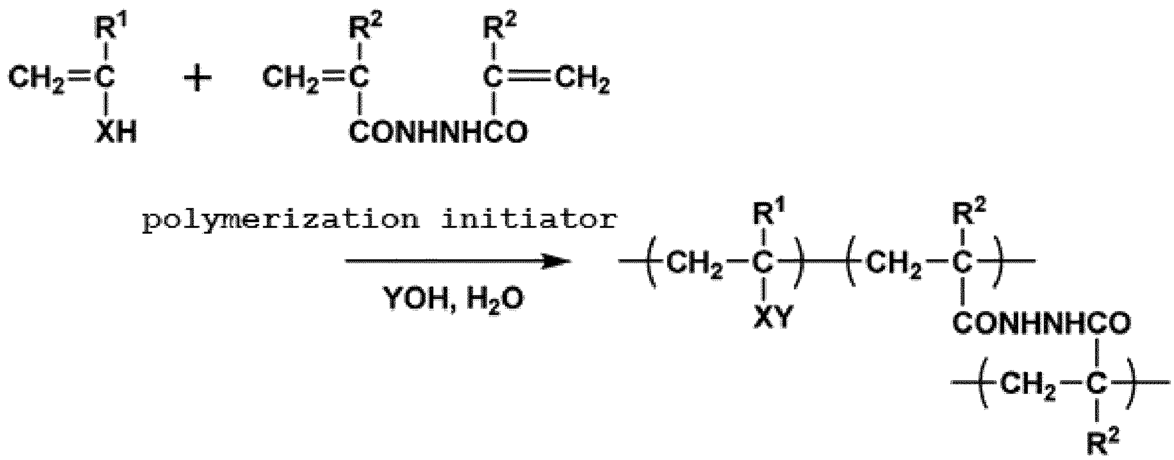


in which $R^1$ and two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is each

independently one or more selected from -$COO^-$, -$SO_3^-$, and - $P(=O)(O^-)_2$; and Y is each independently one or more selected from $Li^+$, $Na^+$, and $K^+$.

Note that, the monomer shown in Formula (3) and the monomer shown in Formula (4) each may be of one type or multiple types, or one may be of one type and the other may be of multiple types.

A polymerizing method may be any polymerizing method to be used for synthesizing, for example, sodium polyacrylate. Specifically, examples of the polymerizing method include aqueous polymerization, reverse-phase suspension polymerization, spray polymerization, droplet polymerization, bulk polymerization, precipitation polymerization, etc. The polymerization method is preferably the aqueous polymerization or the reverse-phase suspension polymerization and more preferably the aqueous polymerization from the viewpoints of easiness in controlling polymerization and water-absorbing performance of a water-absorbing agent.

A polymerization initiator is not particularly limited, but may be a thermal decomposition-based polymerization initiator, a photodecomposition-based polymerization initiator, or a redox-based polymerization initiator in combination with a reducing agent promoting decomposition of these polymerization initiators. Specifically, the polymerization initiator may be a radical polymerization initiator, for example, persulfates such as sodium persulfate, potassium persulfate, and ammonium persulfate; t-butyl hydroperoxide, hydrogen peroxide, etc. Furthermore, an azo-based radical polymerization initiator, for example, an azoamidine compound such as 2,2'-azobis(2-amidinopropane)hydrochloride; a cyclic azoamidine compound such as 2,2'-azobis[2-(3,4,5,6-tetrahydropyrimidine-2-yl)propane]hydrochloride, 2,2'-azobis{2-[1-(2-hydroxyethyl)-2-imidazoline-2-yl]propane}hydrochloride, 2,2'-azobis[2-(2-imidazoline-2-yl)propane]; an azoamide compound such as 2,2'-azobis{2-methyl-N-[1,1-bis(hydroxymethyl)-2-hydroxyethyl]propionamide}, 2,2'-azobis[2-methyl-N-(2-hydroxyethyl)propionamide]; an azocyano compound such as 4,4'-azobis(4-cyanovaleric acid) may be also used as the polymerization initiator. As the polymerization initiator, at least one or two or more polymerization initiators can be selected from the above-mentioned polymerization initiators taking into account of, for example, a polymerization form. Note that, when an oxidative radical polymerization initiator is used, for example, a reducing agent such as sodium sulfite, sodium bisulfite, ferrous sulfate, and L-ascorbic acid may be used therewith.

An amount of the polymerization initiator for use is not particularly limited, but is preferably 0.01% by mole or more, more preferably 0.1% by mole or more, and further preferably 0.5% by mole or more relative to a total amount of the monomers. Furthermore, the amount of the polymerization initiator for use is preferably 10% by mole or less, more preferably 8% by mole or less, further preferably 7% by mole or less relative to a total amount of the monomers.

The polymerization reaction may be initiated by irradiating an active energy ray such as radiation ray, an electron ray, an ultraviolet ray, etc. Furthermore, the polymerization reaction may be initiated by irradiating an active energy ray in combination with the above-mentioned polymerization initiator.

<<Absorbent article>>

An absorbent article according to the present embodiment includes the cross-linked polymer compound used as a water-absorbing material.

The absorbent article can be used as a water-absorbing material in a variety of applications for the purpose of absorbing water such as a disposable diaper, a sanitary article (sanitary napkin), an adult incontinence article (incontinence pad), a hygienic material (hygienic article) such as a pet sheet, a soil water retainer for agricultural and horticultural use, a water sealing agent for industrial use, etc. The above-mentioned cross-linked polymer compound can be used as an alternative to a water-absorbing material for a conventional known absorbent article.

For example, when the disposable diaper or the sanitary article is made as an absorbent article, the disposable diaper and the sanitary article are characterized by including a surface material, a thread for sewing up the surface material, and a water-absorbing material; the water-absorbing material is placed into a space enclosed by the surface material sewn up with the thread; the above-mentioned cross-linked polymer compound is used as the water-absorbing material; and a biodegradable compound or an oxidative-degradable compound is used as the thread.

In the disposable diaper or the sanitary article, since the water-absorbing material is placed into the space enclosed by the surface material sewn up with the thread, the water-absorbing material does not exit the space while the disposable diaper or the sanitary article is in use. On the other hand, when the disposable diaper or the sanitary article after use is contained in, for example, the below-mentioned treatment container, the thread sewing up the surface material and composed of the biodegradable compound or the oxidative-degradable compound decomposes, so that the surface material no longer forms the enclosed space and the water-absorbing material exits the space. The water-absorbing material which has thus exited the space can react with the oxidizing agent within the treatment container to be decomposed into a free polymerized chain and then discharged.

Note that, for the disposable diaper and the sanitary article, in addition to the surface material, the thread, and the water-absorbing material as mentioned above, other materials for exhibiting a variety of functions may be added unless other materials do not prevent the water-absorbing material from contacting with the oxidizing agent.

<<Treatment container>>

A treatment container according to the present embodiment can store the oxidizing agent other than oxygen, and the above-mentioned disposable diaper or sanitary article.

A shape of the treatment container is not particularly limited as long as it can store the oxidizing agent other than oxygen, and the above-mentioned disposable diaper or sanitary article, and may be a cylinder or rectangular tube of which at least one end is closed. The treatment container is preferably a tube of which both ends are closed and of which at least one end is closed by a lid. When opening the lid, the disposable diaper or the sanitary article can be put into the treatment container, where the disposable diaper or the sanitary article contacts with the oxidizing agent (in a form of a solution, as necessary) and decomposes according to the reaction shown by Formula (6) to produce a free polymerized chain.

The oxidizing agent is not particularly limited as long as it is an oxidizing agent other than elemental oxygen, and an oxoacid such as perchloric acid, chloric acid, chlorous acid, hypochlorous acid, perbromic acid, bromic acid, bromous acid, hypobromous acid, periodic acid, iodic acid, iodous acid, hypoiodous acid, nitric acid, and nitrous acid, and any salt or any derivative thereof; elemental chlorine, elemental bromine, elemental iodine, elemental sulfur, and any solution including them; a nitrogen oxide such as nitrogen dioxide and nitric oxide; an oxygen compound such as a superoxide and a peroxide; a transition metal compound such as a transition metal salt and a transition metal complex; or the like may be used alone or as a mixture of two or more thereof. Furthermore, the oxidizing agent may be any one of these oxidizing agents alone or a mixture of two or more of these oxidizing agents with a compound other than these oxidizing agents. Moreover, these oxidizing agents may also be used as the oxidizing agent by using a catalytic amount of any one of these oxidizing agents alone or a mixture of two or more of these oxidizing agents in the presence of elemental oxygen.

A size of the treatment container is not particularly limited and an appropriate size may be selected in consideration of an amount to be treated in a normal facility or the home.

A material of the treatment container is preferably a material that does not or is less likely to deteriorate (corrode, crack, etc.) by the oxidizing agent other than oxygen.

<<Treatment method>>

The treatment method according to the present embodiment includes putting the above-mentioned disposable diaper or sanitary article into the above-mentioned treatment container which stores the oxidizing agent other than oxygen.

Such a treatment method allows the water-absorbing material within the disposable diaper or the sanitary article to be decomposed by the oxidizing agent into a free polymerized chain that does not have a cross-linked structure. The free polymerized chain is water-soluble and thus can be dissolved in the resulting water and easily disposed of as wastewater.

## EXAMPLES

Hereinafter, the present invention will be described in more detail based on Examples and Comparative Examples, but the present invention is not limited to the following Examples.

[Production of cross-linked polymer compound]

Two types of cross-linked polymer compounds differing in a ratio of a monomer represented by [2] were produced by a reaction shown in Formula (8) below. Note that, a compound represented by [4] used as an initiator is represented by Formula (9).

Formula (8):

[Chem. 12]

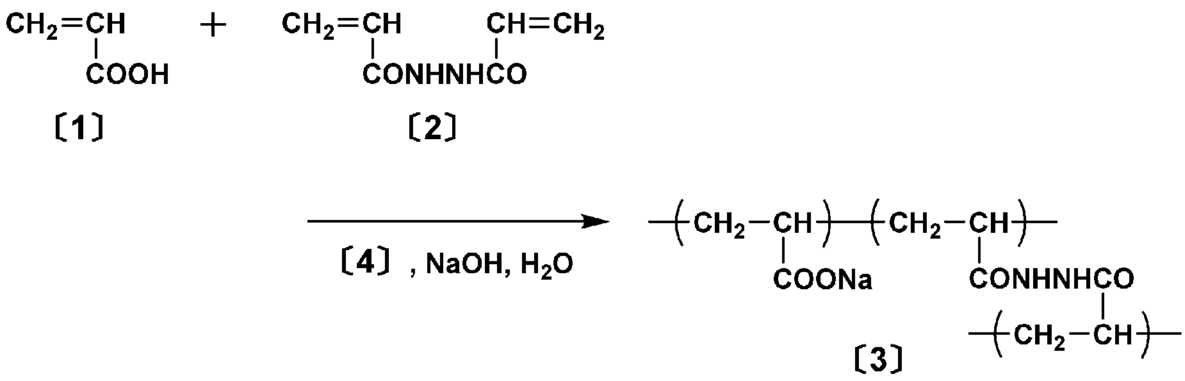


Formula (9):

[Chem. 13]

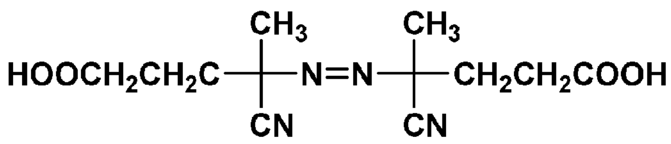

[4]

(Example 1)

A monomer represented by Formula [1] (2.0195 g, 28.03 mmol), a monomer represented by Formula [2] (0.0403 g, 0.288 mmol), NaOH (1.4274 g, 35.69 mmol), 4,4'-azobis(4-cyanovaleric acid) (0.3971 g, 1.417 mmol; V-501 manufactured by FUJIFILM Wako Pure Chemical Corporation; shown in Formula (9) above) were dissolved in 15 mL of water to thereby obtain a raw material aqueous solution. Then, the raw material aqueous solution was performed in a freeze-deaerate process, and heated under an argon atmosphere at 80°C for 19 hours to thereby obtain a gel. This gel was decanted with water three times and then subjected to Soxhlet washing using MeOH for 23 hours to thereby obtain a solid. This solid was vacuum-dried to thereby obtain a cross-linked polymer compound represented by Formula (3) (1.7765 g, yield: 86%) as a white solid.

(Example 2)

A monomer represented by Formula [1] (2.0097 g, 27.89 mmol), a monomer represented by Formula [2] (0.2061 g, 1.471 mmol), NaOH (1.5363 g, 38.41 mmol), 4,4'-azobis(4-cyanovaleric acid) (0.4123 g, 1.471 mmol; V-501 manufactured by FUJIFILM Wako Pure Chemical Corporation) were dissolved in 15 mL of water to thereby obtain a raw material aqueous solution. Then, the raw material aqueous solution was performed in a freeze-deaerate process, and heated under an argon atmosphere at 80°C for 21 hours to thereby obtain a gel. This gel was decanted with water three times and then subjected to Soxhlet washing using MeOH for 22 hours to thereby obtain a solid. This solid was vacuum-dried to thereby obtain a cross-linked polymer compound represented by Formula (3) (2.1536 g, yield: 97%) as a white solid.

[Evaluation of cross-linked polymer compound for water-absorbing property]

The cross-linked polymer compounds of Examples 1 and 2 were dried in a thermostat bath at 70°C for 3 hours, then put into a desiccator with $P_2O_5$ inside and vacuum-dried for 3 days. On the other hand, a polyester tea bag was immersed in 200 mL of distilled water at 25°C for 1 hour, hung for 10 minutes, and then weighed in mass. The cross-linked polymer compound of Example 1 (0.0141 g) or the cross-linked polymer compound of Example 2 (0.0117 g) was put into the tea bag and immersed in 200 mL of distilled water at 25°C. After a predetermined time (designated as "immersion time" in Table 1 below), the tea bag was removed. The post-immersion tea bag containing the cross-linked polymer compound of Example 1 or 2 was hung for 10 minutes and then weighed in mass. A percentage of water absorption (%) of the cross-linked polymer compound was measured according to Expression below.

[Formula 1]

$$\text{Percentage of absorption (\%)} = \frac{\text{Mass of tea bag containing cross-linked polymer after immersion} - \text{Mass of tea bag before immersion}}{\text{Mass of cross-linked polymer compound before immersion}}$$

[Table 1]

| Immersion time / h | Percentage of water absorption (%) | |
|---|---|---|
| | Example 1 | Example 2 |
| 1 | 9796 | 70330 |
| 2 | 9781 | 74990 |
| 3 | 10370 | 78041 |
| 20 | 10745 | 80336 |
| 24 | 10660 | 80520 |

[Evaluation of cross-linked polymer compound for oxidative-degradability]

The cross-linked polymer compound of Example 1 (0.3011 g) was added to 10 mL of a 5% sodium hypochlorite aqueous solution and visually observed while stirring at 25°C. As a result, a uniform solution was obtained in 50 minutes. Furthermore, the cross-linked polymer compound of Example 2 (0.3052 g) was observed in the same manner. As a result, a uniform solution was obtained in 60 minutes. It was confirmed that the cross-linked polymer compounds of Examples 1 and 2 (cross-linked polymer compound represented by Formula [3]) decomposed into a polymer compound represented by [5] as the reaction shown by Formula (10) progressed.

Formula (10):

[Chem. 14]

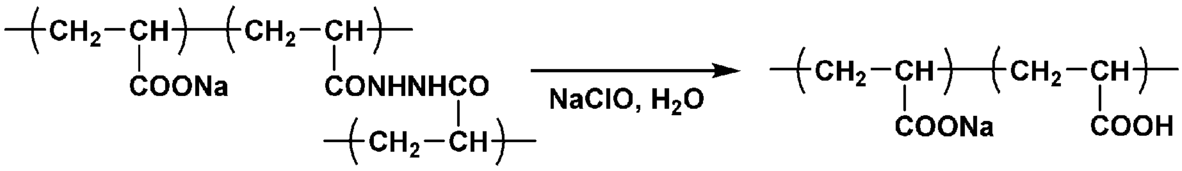


[3]

[5]

## Claims

1. A cross-linked polymer compound comprising:

a skeleton unit shown in Formula (1) below; and
a skeleton unit shown in Formula (2) below, Formula (1):

[Chem. 1]

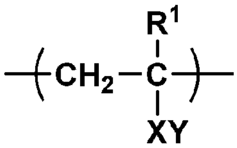

wherein $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; X is one or more selected from $-COO^-$, $-SO_3^-$, and $-P(=O)(O^-)_2$; and Y is one or more selected from $Li^+$, $Na^+$, and $K^+$, and

Formula (2):

[Chem. 2]

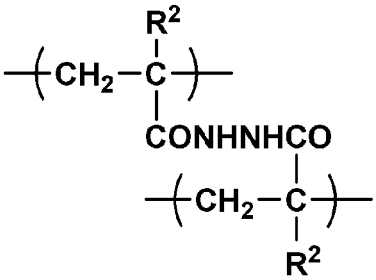

wherein two $R^2$ are each independently one or more selected from hydrogen, an alkyl group, a hydroxy group, an amino group, a mercapto group, a substituted carbonyl group, and any alkyl group having, as a substituent, the above groups.

2. The cross-linked polymer compound according to claim 1, consisting of the skeleton unit shown in Formula (1) and the skeleton unit shown in Formula (2),

wherein a molar ratio of the skeleton unit shown in Formula (1) to the skeleton unit shown in Formula (2) (the skeleton unit shown in Formula (1):the skeleton unit shown in Formula (2)) is 90:10 to 99.9999:0.0001.

**3.** The cross-linked polymer compound according to claim 1 or 2, wherein the part $R^1$ in Formula (1) and the two $R^2$ are H, the part X is $-COO^-$, and the part Y is $Na^+$.

**4.** The cross-linked polymer compound according to any one of claims 1 to 3, being a random copolymer.

**5.** An absorbent article comprising:

the cross-linked polymer compound according to any one of claims 1 to 4, the cross-linked polymer compound being used as a water-absorbing material.

**6.** A disposable diaper comprising:

the cross-linked polymer compound according to any one of claims 1 to 4, the cross-linked polymer compound being used as a water-absorbing material.

**7.** A disposable diaper comprising:

a surface material;
a thread for sewing up the surface material; and
a water-absorbing material, wherein
the water-absorbing material is placed into a space enclosed by the surface material sewn up with the thread,
the cross-linked polymer compound according to any one of claims 1 to 4 is used as the water-absorbing material, and
a biodegradable compound or an oxidative-degradable compound is used as the thread.

**8.** A sanitary article comprising:

the cross-linked polymer compound according to any one of claims 1 to 4, the cross-linked polymer compound being used as a water-absorbing material.

**9.** A sanitary article comprising:

a surface material;
a water-absorbing material; and
a thread for sewing up the surface material, wherein
the water-absorbing material is placed into a space enclosed by the surface material sewn up with the thread,
the cross-linked polymer compound according to any one of claims 1 to 4 is used as the water-absorbing material, and
a biodegradable compound or an oxidative-degradable compound is used as the thread.

**10.** A treatment container capable of storing:

an oxidizing agent other than oxygen; and
the disposable diaper according to claim 6 or 7 or the sanitary article according to claim 8 or 9.

**11.** A treatment method comprising:
putting the disposable diaper according to claim 6 or 7 or the sanitary article according to claim 8 or 9 into the treatment container according to claim 10 which stores an oxidizing agent of oxygen.

**12.** A method for producing a cross-linked polymer compound comprising:
copolymerizing a monomer shown in Formula (3) below and a monomer shown in Formula (4) below,

Formula (3):

[Chem. 3]

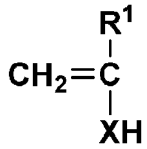

wherein $R^1$ is one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group; and X is each independently one or more selected from $-COO^-$, $-SO_3^-$, and $-P(=O)(O^-)_2$, and

Formula (4):

[Chem. 4]

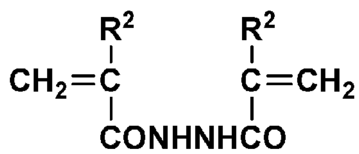

wherein two $R^2$ are each independently one or more selected from hydrogen, an alkyl group, a hydroxy group, an amino group, a mercapto group, a substituted carbonyl group, and any alkyl group having, as a substituent, one or more selected from a hydroxy group, an amino group, a mercapto group, and a substituted carbonyl group.

## INTERNATIONAL SEARCH REPORT

International application No. PCT/JP2019/051328

A. CLASSIFICATION OF SUBJECT MATTER
Int. Cl. C08F220/04(2006.01)i, A61F13/53(2006.01)i, A61L15/22(2006.01)i
FI: C08F220/04, A61F13/53 300, A61L15/22 200

According to International Patent Classification (IPC) or to both national classification and IPC

B. FIELDS SEARCHED

Minimum documentation searched (classification system followed by classification symbols)
Int. Cl. C08F220/04, A61F13/53, A61L15/22

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 11-123885 A (TORAY INDUSTRIES, INC.) 11 May 1999, paragraphs [0042]-[0050], examples 8, 14 | 12<br>1-11 |
| A | JP 2001-507736 A (KIMBERLY-CLARK WORLDWIDE, INC.) 12 June 2001 | 1-12 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12.03.2020 | 24.03.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2019/051328

| Patent Documents referred to in the Report | Publication Date | Patent Family | Publication Date |
|---|---|---|---|
| JP 11-123885 A | 11.05.1999 | US 6194122 B1<br>column 5, line 14 to column 6, line 45, examples 8, 14<br>EP 897795 A1<br>CA 2245304 A1 | |
| JP 2001-507736 A | 12.06.2001 | US 5969052 A<br>WO 1998/029461 A1<br>EP 950072 A1<br>AU 5381298 A<br>IL 130379 D<br>BR 9715021 A<br>CA 2274679 A<br>CN 1248982 A<br>KR 10-0547067 B1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H11315147 A